# EUROPÄISCHE PATENTSCHRIFT

(11) **EP 1 514 563 B1**
(45) Veröffentlichungstag und Bekanntmachung des Hinweises auf die Patenterteilung: **29.03.2006**
(21) Anmeldenummer: 04018327.9
(22) Anmeldetag: 02.08.2004
(51) Int. Cl.: A61M 1/36, A61M 1/14, A61L 2/00

(54) **Desinfektions- bzw. Reinigungsmittelanschlusseinrichtung**
Connection device for sterilant or cleaning agent
Dispositif de connexion pour stérilisant ou produit de nettoyage

(30) Priorität: 03.09.2003 DE 10340648
(43) Veröffentlichungstag der Anmeldung: 16.03.2005
(73) Patentinhaber: Fresenius Medical Care Deutschland GmbH, 61352 Bad Homburg (DE)
(72) Erfinder: Brandl, Matthias, Dr., 97631 Bad Königshofen (DE); Wolter, Elmar, 97230 Estenfeld (DE)
(74) Vertreter: Laufhütte, Dieter

(56) Entgegenhaltungen:
- EP-A- 1 437 547
- DE-C- 4 321 008
- GB-A- 1 216 628
- US-A- 5 656 027

## Beschreibung

Die Erfindung betrifft eine Desinfektionsmittel- bzw. Reinigungsmittelanschlußeinrichtung für Geräte, die einen Raum zur Aufnahme oder zum Transport von Flüssigkeiten aufweisen, mit einer Verbindungsleitung zur Verbindung des Flüssigkeitsraums mit einem Desinfektionsmittel- bzw. Reinigungsmittelbehälter.

Zur Reinigung medizinischer Geräte, die einen Flüssigkeitskreislauf für medizinische Flüssigkeiten aufweisen, werden Desinfektionsmittel bzw. spezielle Reinigungsmittel durch den Flüssigkeitskreislauf geleitet. Wenn ein Desinfektionsmittelbehälter an eine solche medizinische Vorrichtung, z. B. ein Dialysegerät, angeschlossen ist, darf kein Desinfektionsmittel ungewollt in die Vorrichtung laufen. Andererseits darf während des normalen Betriebes des medizinischen Gerätes keine Flüssigkeit aus der medizinischen Vorrichtung in den Desinfektionsmittelbehälter fließen und das Desinfektionsmittel in unkontrollierter Weise verdünnen.

Bei bekannten Lösungen werden hierzu entsprechend angeordnete Rückschlagventile und gesteuerte Magnetventile eingesetzt. In DE 43 21 008 C1 ist zur Überwachung der Dichtigkeit ein Puffervolumen in der Versorgungsleitung vorgesehen, in dem ein anderer Druck eingestellt wird als in der übrigen Versorgungsleitung. Der Druck des Puffervolumens wird überwacht. Ändert sich der Druck in dem Puffervolumen, ist dies gleichbedeutend mit einer Leckage eines der das Puffervolumen abschließenden Ventile.

Es ist die Aufgabe der vorliegenden Erfindung, eine Desinfektionsmittel- bzw. Reinigungsmittelanschlußeinrichtung anzugeben, die zum einen einfach und kostengünstig realisierbar ist und zum anderen eine hohe Betriebssicherheit, insbesondere für medizinische Anwendungen gewährleistet. Diese Aufgabe wird mit einer Desinfektionsmittel- bzw. Reinigungsmittelanschlußeinrichtung mit den Merkmalen des Anspruches 1 gelöst. Die Unteransprüche sind auf vorteilhafte Ausgestaltungen gerichtet. Die Aufgabe wird zudem mit einem medizinischen Gerät mit den Merkmalen des Anspruches 6, einer RO-Anlage (Umkehr (Reverse) Osmose Anlage) mit den Merkmalen des Anspruches 5 oder einem Dialysegerät mit den Merkmalen des Anspruchs 4 gelöst. Vorteilhafte Verwendungen sind Gegenstand des Anspruches 7.

Im folgenden werden die Effekte der Erfindung für medizinische Geräte bzw. medizinische Flüssigkeiten beschrieben, bei denen der sichere Betrieb besonders vorteilhaft ist. Die Erfindung kann jedoch ebenso für nicht medizinische Geräte zum Einsatz kommen.

Die erfindungsgemäße Desinfektionsmittel- bzw. Reinigungsmittelanschlußeinrichtung weist eine Verbindungsleitung des Flüssigkeitsraumes, der zur Aufnahme bzw. zum Transport von medizinischen Flüssigkeiten vorgesehen ist, mit einem Desinfektionsmittelbehälter auf, die einen Bereich aufweist, der einen Aufnahmeraum bildet. Im oberen Bereich des Aufnahmeraums ist ein Verschlußelement zum Anschluß des Aufnahmeraums an den Flüssigkeitsraum vorgesehen. Weiterhin weist die erfindungsgemäße Desinfektionsmittel- bzw. Reinigungsmittelanschlußeinrichtung einen Kanal auf, der unten in den Aufnahmeraum mündet. Der Kanal weist einen siphonartigen Abschnitt auf, der von der Mündung ausgehend einen nach oben weisenden Teil, einen oberen Teil und einen nach unten weisenden Teil aufweist, wobei der nach unten weisende Teil ein Ende hat, das tiefer liegt als der Boden des Aufnahmeraums. Weiterhin weist die erfindungsgemäße Desinfektionsmittel- bzw. Reinigungsmittelanschlußeinrichtung einen Flüssigkeitssensor in dem Aufnahmeraum auf, der auf einem Niveau angeordnet ist, das tiefer als der obere Teil des Siphonbereiches des Kanals ist und höher als die Kanaleinmündung in den Aufnahmeraum liegt.

Wenn im folgenden von Desinfektionsmitteln die Rede ist, so sollen damit allgemein auch andere Reinigungsmittel gemeint sein.

Bei dem Aufnahmeraum kann es sich z. B. um einen starren Behälter, um ein Rohrsegment oder um ein Schlauchsegment handeln. Das Verschlußelement kann z. B. ein Ventil oder eine flußunterbrechende Pumpe umfassen. Im folgenden wird der Einfachheit halber der Fall eines Ventiles als Verschlußelement angenommen.

Während des Normalbetriebes ist der Aufnahmeraum mit Hilfe des Ventiles an den Flüssigkeitsraum der medizinischen Vorrichtung angeschlossen. Das untere Ende des Kanals ist in einem Desinfektionsmittel- bzw. Reinigungsmittelkanister eingeführt. Während des bestimmungsgemäßen Betriebes der medizinischen Vorrichtung ist das Ventil geschlossen, so daß zum einen keine medizinische Flüssigkeit aus der medizinischen Vorrichtung in den Desinfektionsmittelbehälter eintreten kann und zum anderen kein Desinfektionsmittel in die medizinische Vorrichtung gelangen kann. Im Falle eines Fehlers des Ventils leckt medizinische Flüssigkeit durch das Ventil in den Aufnahmeraum. Dieser wird durch diese einleckende Flüssigkeit langsam gefüllt, bis der Flüssigkeitssensor die eintretende Flüssigkeit detektiert und z. B. ein Alarmsignal ausgibt und ggf. die Behandlung unterbricht.

Aufgrund des siphonartigen Anschlusses des Aufnahmeraums an dem Desinfektionsmittelbehälter wird dennoch wirksam verhindert, daß medizinische Flüssigkeit, die in den Aufnahmeraum eintritt, direkt in den Desinfektionsmittelbehälter fließt und darin das Desinfektionsmittel in unkontrollierter Weise verdünnt bzw. verändert.

Sollte während des bestimmungsgemäßen Betriebes aufgrund eines Fehlers das Ventil zwischen Flüssigkeitsraum und Desinfektionsmittelbehälter geöffnet sein und die Pumpe, die bestimmungsgemäß zur Einleitung des Desinfektionsmittels in die medizinische Vorrichtung vorgesehen ist, in Betrieb sein, so tritt Desinfektionsmittel aus dem Desinfektionsmittelbehälter durch den siphonartigen Kanal in den Aufnahmeraum unkontrolliert ein. Der Flüssigkeitssensor wird benetzt, wodurch der fehlerhafte Zustand detektiert wird.

Im Reinigungsbetrieb wird durch den Kanal, den Aufnahmeraum und das jetzt geöffnete Ventil Reinigungsflüssigkeit in die medizinische Vorrichtung gepumpt. Nach Abschluß des Reinigungsvorganges wird das Desinfektionsmittel wieder abgelassen. Es läuft dabei durch das geöffnete Ventil in den Aufnahmeraum und füllt diesen bis zu einem Füllstand, der dem oberen Bereich des siphonartigen Abflußkanals entspricht. Jetzt wird über diesen Siphon die Flüssigkeit über die jetzt kommunizierenden Röhren in den Desinfektionsmittelkanister abgeleitet. Da der siphonartige Kanal ganz unten in den Aufnahmeraum mündet, ist sichergestellt, daß der gesamte Aufnahmeraum auf diese Weise nach dem Prinzip der kommunizierenden Röhren entleert wird.

Die erfindungsgemäße Desinfektionsmittel- bzw. Reinigungsmittelanschlußeinrichtung für medizinische Geräte gewährleistet also höchste Sicherheit und einfache Handhabung. Im bestimmungsgemäßen Betrieb der medizinischen Vorrichtung wird sicher detektiert, wenn eine Leckage des Absperrventils zwischen Flüssigkeitsraum und Desinfektionsmittelbehälter auftritt. Nach einer Reinigung der medizinischen Vorrichtung mit Desinfektionsmittel wird durch den besonders gestalteten siphonartigen Abfluß gewährleistet, daß das gesamte Desinfektionsmittel aus dem Aufnahmeraum und der Verbindung zum Desinfektionsmittelbehälter in den Desinfektionsmittelbehälter abgeführt wird. Die erfindungsgemäße Vorrichtung stellt also sicher, daß weder die medizinische Flüssigkeit durch Desinfektionsmittel geschädigt werden kann, noch daß das Desinfektionsmittel in dem Kanister auf unkontrollierte Weise mit medizinischer Flüssigkeit versetzt bzw. verdünnt wird.

Trotzdem kann der Desinfektionsmittelbehälter immer mit der medizinischen Vorrichtung verbunden bleiben.

Einen unkontrollierten Zufluß von Desinfektionsmittel in die medizinische Vorrichtung bei einer fehlerhaften Durchschaltung einer Pumpe bzw. des Ventils wird durch den unkontrollierten Flüssigkeitsfluß an dem Flüssigkeitssensor detektiert.

Weist der Aufnahmeraum gemäß einer bevorzugten Ausführungsform einen größeren Querschnitt als die sich anschließenden Teile der Verbindungsleitung auf, so ist ein besonders sicherer Betrieb möglich.

Eine erfindungsgemäße Desinfektionsmittel- bzw. Reinigungsmittelanschlußeinrichtung mit einer noch weiter erhöhten Sicherheitsstufe weist vorzugsweise zusätzlich ein Lüftungsventil im oberen Bereich des Aufnahmeraums auf. Im bestimmungsgemäßen Betrieb der medizinischen Vorrichtung, bei dem das Absperrventil zwischen Desinfektionsmittelbehälter und Flüssigkeitsraum für die medizinische Flüssigkeit geschlossen sein sollte, ist dieses Lüftungsventil des Aufnahmeraumes geöffnet. Sollte aufgrund eines Fehlers das Absperrventil zwischen Flüssigkeitsraum und Desinfektionsmittelbehälter geöffnet sein und die Pumpe, die zur Einleitung von Desinfektionsmittel in die medizinische Vorrichtung vorgesehen ist, in Betrieb sein, so wird mit Hilfe dieses geöffneten Lüftungsventils verhindert, daß Desinfektionsmittel in die medizinische Vorrichtung eingepumpt wird. Statt dessen wird nur Luft durch das geöffnete Lüftungsventil und den Aufnahmeraum in die medizinische Vorrichtung angesaugt. Während des Reinigungsbetriebes wird das Lüftungsventil geschlossen, so daß ein Ansaugen der Desinfektionsflüssigkeit aus dem Behälter in die medizinische Vorrichtung möglich ist.

Als Flüssigkeitssensor können unterschiedliche Sensoren eingesetzt werden. Zum Beispiel kann ein Leitfähigkeitssensor verwendet werden, der die Leitfähigkeit seines umgebenden Mediums überwacht und somit bei Benetzung mit Flüssigkeit ein Signal aussendet. Bei einer besonders vorteilhaften Ausführungsform wird ein optischer Flüssigkeitssensor eingesetzt, der es ermöglicht, auch Flüssigkeit in äußerst geringer oder ohne jede Leitfähigkeit zu überwachen. Die Funktion des optischen Sensors läßt sich mit einem periodischen Funktionstest einfach und automatisch überprüfen.

Besonders vorteilhaft läßt sich die erfindungsgemäße Desinfektionsmittel- bzw. Reinigungsmittelanschlußeinrichtung bei medizinischen Geräten verwenden, bei denen medizinische Flüssigkeiten auf hoch sensible Weise in Flüssigkeitskreisläufen transportiert werden müssen, wie z. B. Dialysevorrichtungen oder RO-Anlagen. Unter RO-Anlage ist dabei eine Umkehr (Reverse) Osmose Anlage zu verstehen.

Eine Ausführungsform der erfindungsgemäßen Desinfektionsmittel- bzw. Reinigungsmittelanschlußeinrichtung wird anhand der beiliegenden schematischen Figur im Detail erläutert.

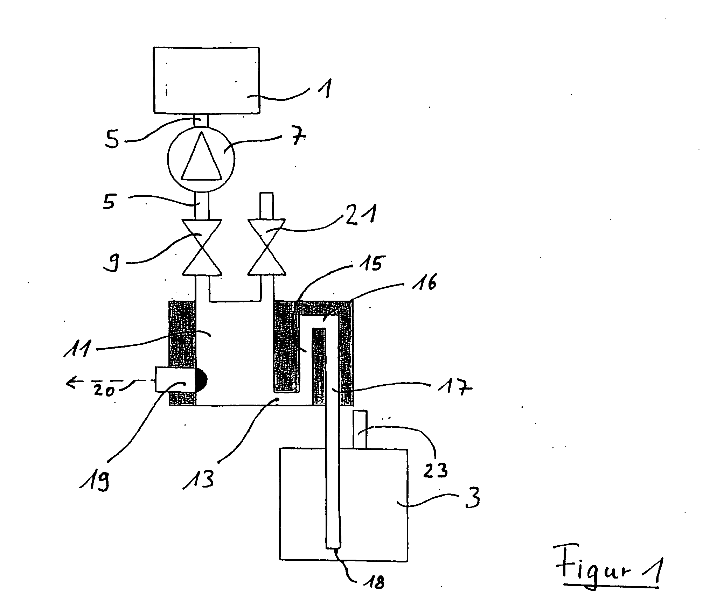

1 bezeichnet schematisch eine medizinische Vorrichtung, die einen Flüssigkeitskreislauf umfaßt, z. B. einen Dialyseapparat. Der Dialyseapparat ist über eine Verbindungsleitung 5 angeschlossen, die eine Pumpe 7 aufweist. Ein Absperrventil 9 verbindet das Dialysegerät 1 mit einem Aufnahmeraum 11. Alternativ kann das Absperrventil 9 wegfallen und die Pumpe 7 als flußunterbrechende Pumpe ausgestaltet sein. Der Aufnahmeraum 11 hat vorzugsweise einen größeren Querschnitt als die Leitung 5. Direkt benachbart zum Boden des Aufnahmeraums 11 mündet ein Kanal durch die Kanalmündung 13 in den Aufnahmeraum 11 ein, der eine siphonartige Ausgestaltung mit einem nach oben weisenden Bereich 15, einem oberen Teil 16 und einem nach unten weisenden Bereich 17 hat. Der nach unten weisende Bereich 17 reicht in einen Desinfektionsmittelkanister 3. Dabei ist das Ende 18 des nach unten weisenden Bereiches 17 im unteren Bereich des Kanisters 3 angeordnet und liegt tiefer als der Boden des Aufnahmeraums 11. Im unteren Bereich des Aufnahmeraums, jedoch oberhalb der Kanalmündung 13 ist ein optischer Flüssigkeitssensor 19 vorgesehen. In der Figur der Übersichtlichkeit halber nicht dargstellt sind Zuführleitungen zu dem Flüssigkeitssensor 19 zur Sicherstellung dessen Energieversorgung. 20 deutet schematisch eine Signalleitung an, mit deren Hilfe das Signal des Flüssigkeitssensors 19 an eine Auswerteeinrichtung oder eine Warneinrichtung gegeben werden kann.

Oben am Aufnahmeraum 11 ist ein Lüftungsventil 21 vorgesehen. Schließlich weist der Desinfektionsmittelbehälter 3 ein Entlüftungsventil 23 auf.

Die erfindungsgemäße Ausführungsform wird wie folgt eingesetzt. Während des bestimmungsgemäßen Gebrauches der Dialyseeinrichtung 1 ist die Pumpe 7 abgeschaltet und das Ventil 9 geschlossen. Das Lüftungsventil 21 ist geöffnet. Die Dialyseeinrichtung arbeitet in an sich bekannter Weise zur Reinigung des Blutes eines Patienten. Desinfektionsmittel, das während des bestimmungsgemäßen Gebrauches der Dialyseeinrichtung 1 nicht im Einsatz ist, befindet sich im Desinfektionsmittelbehälter 3. Der Aufnahmeraum 11 ist leer.

Sollte es zu einer Fehlfunktion oder Undichtigkeit des Ventils 9 kommen, so leckt die medizinische Flüssigkeit aus der Dialyseeinrichtung 1 durch das Ventil 9 hindurch in den Aufnahmeraum 11. Aufgrund der siphonartigen Ausgestaltung des Abflußkanals 15, 16, 17 füllt sich zunächst der Aufnahmeraum 11 mit der medizinischen Flüssigkeit. Sobald der Flüssigkeitsstand den Flüssigkeitssensor 19 erreicht hat, gibt dieser über die Signalleitung 20 ein Signal an eine in der Figur nicht dargestellte Alarmeinrichtung, z. B. einen akustischen oder optischen Signalgeber. Es kann auch vorgesehen sein, dass das Signal zur automatischen Unterbrechung der medizinischen Behandlung eingesetzt wird. Durch den siphonartigen Aufbau der Verbindung 13, 15, 16, 17 des Aufnahmeraums 11 mit dem Desinfektionsmittelbehälter 3 ist sichergestellt, daß die medizinische Flüssigkeit, die durch das Ventil 9 in den Aufnahmeraum 11 leckt, nicht in den Desinfektionsmittelbehälter 3 gelangen kann. Das darin enthaltene Desinfektionsmittel wird also durch die medizinische Flüssigkeit nicht beeinträchtigt.

Ist während der bestimmungsgemäßen Verwendung der Dialyseeinrichtung 1 bei der hier beschriebenen Ausführung das Lüftungsventil 21 jedoch geschlossen und sollte es aufgrund einer Fehlfunktion oder eines Bedienungsfehlers während der Dialyse ungewollt zu einer Öffnung des Ventils 9 und/oder eines Einschaltens der Pumpe 7 kommen, so wird Desinfektionsmittel durch den Kanal 15, 16, 17 unkontrolliert in den Aufnahmeraum 11 eintreten. Der Flüssigkeitssensor 19 schlägt an und ein entsprechendes Alarmsignal wird über die Signalleitung 20 abgeben. Aufgrund des Aufnahmeraums 11 und der Einmündung des Kanals 13 im unteren Bereich des Aufnahmeraums ist sichergestellt, daß keine Desinfektionsflüssigkeit in das Dialysegerät 1 gelangt. Weiterhin ist während des bestimmungsgemäßen Gebrauches der Dialysevorrichtung 1 das Lüftungsventil 21 geöffnet, so daß konstrukiv das Ansaugen von Desinfektionsmittel in die Dialyseeinrichtung 1 durch die möglicherweise fehlerhaft eingeschaltete Pumpe 7 weitgehend ausgeschlossen ist. Stattdessen gelangt nur Luft durch das Lüftungsventil 21 und den Aufnahmeraum 11 in die Dialyseeinrichtung 1.

Nach der Dialysebehandlung muß die Dialyseeinrichtung 1 gereinigt werden. Dazu wird das Ventil 9 geöffnet und die Pumpe 7 eingeschaltet. Das Lüftungsventil 21 wird geschlossen. Desinfektionsmittel wird aus dem Desinfektionsmittelbehälter 3 durch den Kanal 15, 16, 17 und durch den Aufnahmeraum 11 in die Dialyseeinreichung 1 gepumpt und dort zur Reinigung eingesetzt. Nach Abschluß der Reinigung wird die Pumpe 7 abgeschaltet und das Ventil 9 geschlossen. Der Aufnahmerahmen 11 sowie der siphonartige Abfluss 13, 15, 16, 17 sind mit Desinfektionsmittel gefüllt. Durch Öffnen des Lüftungsventiles 21 fließt das Desinfektionsmittel aus dem Aufnahmeraums 11 und dem siphonartigen Abluss in den Desinfektionsbehälter ab. Da dieser und das Ende 18 des siphonartigen Abflusses unterhalb des Aufnahmeraums angeordnet sind, wird der Kanal 15, 16, 17 und der Aufnahmeraum 11 nach dem Prinzip der kommunizierenden Röhren durch den Siphonabfluß 15, 16, 17 vollständig entleert.

Die erfindungsgemäße Vorrichtung ist kostengünstig und durch einfache Elektronik zu realiseren. Das ungewollte Ansaugen toxischer Flüssigkeit wird ausgeschlossen. Sowohl der Leckagefluß vom Desinfektionsmittelkanister zu der medizinischen Vorrichtung als auch der Leckagefluß von der medizinischen Vorrichtung in den Desinfektionsmittelkanister zurück wird sicher detektiert. Die erfindungsgemäße Vorrichtung ermöglicht die Detektion geringster Leckageflüsse, z. B. kleiner als 1 ml pro Tag.

## Patentansprüche

1. Desinfektions- bzw. Reinigungsmittelanschlußeinrichtung für Geräte, die einen Raum zur Aufnahme oder zum Transport von Flüssigkeiten aufweisen, mit einer Verbindungsleitung zur Verbindung des Flüssigkeitsraumes (1) mit einem Desinfektions- bzw. Reinigungsmittelbehälter (3), insbesondere für medizinische Geräte, wobei die Verbindungsleitung folgendes aufweist:
- einen Aufnahmeraum (11) mit einem Querschnitt vorzugsweise größer als der Querschnitt der sich an den Aufnahmeraum (11) anschließenden Teile der Verbindungsleitung,
- ein Verschlußelement (9) am oberen Bereich des Aufnahmeraums (11) zu dessen Anschluß an den Flüssigkeitsraum (1),
- einen Kanal (15, 16, 17), der unten in den Aufnahmeraum (11) mündet und einen siphonartigen Abschnitt (15, 16, 17) aufweist, der von seiner Mündung (13) in den Aufnahmeraum ausgehend einen nach oben weisenden Teil (15), einen oberen Bereich (16) und einen nach unten weisenden Teil (17) umfaßt, wobei der nach unten weisende Teil ein Ende (18) aufweist, das tiefer liegt als der Boden des Aufnahmeraums (11), und
- einen Flüssigkeitssensor (19) in dem Aufnahmeraum (11), der in einem Niveau angeordnet ist, das tiefer als der obere Teil (16) des Siphonabschnittes (15, 16, 17) und höher als die Kanaleinmündung (13) des Kanals in dem Aufnahmeraum (11) liegt.

2. Desinfektions- bzw. Reinigungsmittelanschlußeinrichtung nach Anspruch 1 mit einem Lüftungsventil (21) am oberen Teil des Aufnahmeraums (11).

3. Desinfektions- bzw. Reinigungsmittelanschlußeinrichtung nach einem der Ansprüche 1 oder 2, bei der der Flüssigkeitssensor einen optischen Sensor umfaßt.

4. Dialysegerät mit einer Desinfektionsmittel- bzw. Reinigungsmittelanschlußeinrichtung nach einem der Ansprüche 1 bis 3.

5. RO-Anlage mit einer Desinfektionsmittel- bzw. Reinigungsmittelanschlußeinrichtung nach einem der Ansprüche 1 bis 3.

6. Medizinisches Gerät mit einer Desinfektionsmittel- bzw. Reinigungsmittelanschlusseinrichtung nach einem der Ansprüche 1 bis 3.

7. Verwendung einer Desinfektionsmittel- bzw. Reinigungsmittelanschlußeinrichtung nach einem der Ansprüche 1 bis 3 für die Desinfektion bzw. Reinigung von medizinischen Geräten, insbesondere Dialysegeräten oder RO-Anlagen.

## Claims

1. A disinfecting or cleaning agent connection device for apparatuses which have a chamber for receiving or transporting fluids, comprising a connection conduit for connecting the fluid chamber (1) to a disinfecting or cleaning agent container (3), in particular for medical apparatuses, wherein the connection conduit has the following:
- a receiving chamber (11) of a cross-section which is preferably larger than the cross-section of the parts of the connection conduit, which connect to the receiving chamber (11),
- a closure element (9) at the upper region of the receiving chamber (11) for the connection thereof to the fluid chamber (1),
- a passage (15, 16, 17) which opens at the bottom into the receiving chamber (11) and has a siphon-like portion (15, 16, 17) which, from its mouth opening (13) into the receiving chamber, has an upwardly pointing part (15), an upper region (16) and a downwardly pointing part (17), wherein the downwardly pointing part has an end (18) which is lower than the bottom of the receiving chamber (11), and
- a fluid sensor (19) in the receiving chamber (11) which is arranged at a level which is lower than the upper part (16) of the siphon portion (15, 16, 17) and higher than the passage mouth opening (13) of the passage in the receiving chamber (11).

2. A disinfecting or cleaning agent connection device according to claim 1 comprising a ventilation valve (21) at the upper part of the receiving chamber (11).

3. A disinfecting or cleaning agent connection device according to one of claims 1 and 2 wherein the fluid sensor includes an optical sensor.

4. A dialysis apparatus with a disinfecting or cleaning agent connection device according to one of claims 1 to 3.

5. A reverse osmosis installation with a disinfecting or cleaning agent connection device according to one of claims 1 to 3.

6. A medical apparatus with a disinfecting or cleaning agent connection device according to one of claims 1 to 3.

7. Use of a disinfecting or cleaning agent connection device according to one of claims 1 to 3 for disinfecting or cleaning medical apparatuses, in particular dialysis apparatuses or reverse osmosis installations.

## Revendications

1. Dispositif de raccordement à un produit désinfectant ou nettoyant pour des appareils, qui comportent un espace pour la réception ou le transport de liquides, comportant une conduite de raccordement pour le raccordement de l'espace contenant le liquide (1) à un réservoir contenant le produit désinfectant ou nettoyant (3), en particulier pour des appareils médicaux, la conduite de raccordement comportant ce qui suit:
- un espace de réception (11) avec une section transversale de préférence supérieure à la section transversale des parties de la conduite de raccordement se rattachant à l'espace de réception (11),
- un élément de fermeture (9) sur la zone supérieure de l'espace de réception (11) pour son raccordement à l'espace contenant le liquide (1),
- un canal (15, 16, 17) qui débouche en bas dans l'espace de réception (11) et comporte une section à mode de siphon (15, 16, 17) qui englobe, à partir de son embouchure (13) dans l'espace de réception, une partie orientée vers le haut (15), une zone supérieure (16) et une partie orientée vers le bas (17), la partie orientée vers le bas comportant une extrémité (18) qui se situe plus bas que le plancher de l'espace de réception (11) et
- un détecteur de liquide (19) dans l'espace de réception (11) qui est disposé à un niveau qui est plus bas que la partie supérieure (16) de la section à siphon (15, 16, 17) et plus haut que l'embouchure (13) du canal dans l'espace de réception (11).

2. Dispositif de raccordement à un produit désinfectant ou nettoyant selon la revendication 1 comportant une soupape d'aération (21) sur la partie supérieure de l'espace de réception (11).

3. Dispositif de raccordement à un produit désinfectant ou nettoyant selon l'une quelconque des revendications 1 ou 2 dans lequel le détecteur de liquide comporte un détecteur optique.

4. Appareil de dialyse comportant un dispositif de raccordement à un produit désinfectant ou nettoyant selon l'une quelconque des revendications 1 à 3.

5. Installation OI comportant un dispositif de raccordement à un produit désinfectant ou nettoyant selon l'une quelconque des revendications 1 à 3.

6. Appareil médical comportant un dispositif de raccordement à un produit désinfectant ou nettoyant selon l'une quelconque des revendications 1 à 3.

7. utilisation d'un dispositif de raccordement à un produit désinfectant ou nettoyant selon l'une quelconque des revendications 1 à 3 pour la désinfection ou le nettoyage d'appareils médicaux, en particulier d'appareils de dialyse ou d'installations OI.
